(19) Europäisches Patentamt
European Patent Office
Office européen des brevets

(11) Veröffentlichungsnummer: **0 348 351**
**A1**

(12) # EUROPÄISCHE PATENTANMELDUNG

(21) Anmeldenummer: **89810450.0**

(22) Anmeldetag: **13.06.89**

(51) Int. Cl.⁴: **C 07 D 307/32**
C 07 D 307/68, A 01 N 43/08

(30) Priorität: **22.06.88 CH 2391/88**

(43) Veröffentlichungstag der Anmeldung:
**27.12.89 Patentblatt 89/52**

(84) Benannte Vertragsstaaten:
**AT BE CH DE ES FR GB GR IT LI LU NL**

(71) Anmelder: **CIBA-GEIGY AG**
**Klybeckstrasse 141**
**CH-4002 Basel (CH)**

(72) Erfinder: **Sutter, Marius, Dr.**
**Klingental 5**
**CH-4058 Basel (CH)**

**Buser, Hans-Peter, Dr.**
**General Guisan Strasse 37**
**CH-4144 Arlesheim (CH)**

**Pugin, Benoit, Dr.**
**Wasserhausweg 16**
**CH-4142 Münchenstein (CH)**

**Eckhardt, Wolfgang, Dr.**
**Breslauerstrasse 14**
**CH-7850 Lörrach (CH)**

**Spindler, Felix, Dr.**
**Dullikerstrasse 392**
**CH-4656 Starrkirch-Wil (CH)**

Patentanspruch für folgenden Vertragsstaat: ES

(54) **Verfahren zur Herstellung von substituierten Aniliden.**

(57) Ein mehrstufiges Verfahren zur Herstellung von (aS;1'R)-3-[N-(Acyl)-N-(3-chlorphenyl)]-amino-tetrahydro-2-furanonen der Formel I

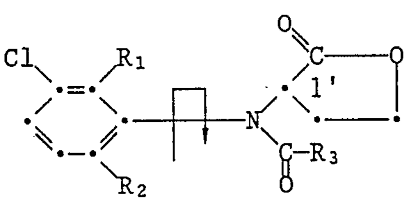 (I)

worin
$R_1$ und $R_2$ voneinander unabhängig Methyl oder Aethyl und $R_3$ Methyl, Chlormethyl, Methoxymethyl oder Cyclopropyl bedeuten wird beschrieben.
Dabei wird ein α-Phenylamino-γ-butyrolacton der Formel V zu einem 3-(Phenylamino)-2,5-dihydro-2-furanon der Formel IV

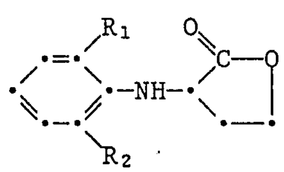
(V)

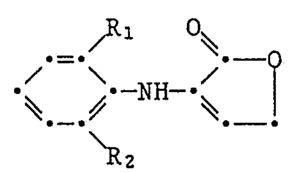
(IV)

oxidiert, dieses mit einem Carbonsäurehalogenid oder Carbonsäureanhydrid der Formel VI

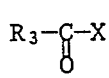
(VI)

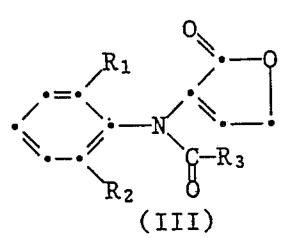
(III)

worin X Chlor, Brom oder den Rest $R_3COO$- bedeutet, zu einem 3-[N-(Acyl)-N-(phenyl)]-amino-2,5-dihydro-2-furanon der Formel III acyliert, das resultierende Produkt in Gegenwart von

EP 0 348 351 A1

enantioselektiv wirkenden Katalysatoren mit Wasserstoff zu einem (1'R) 3-[N-(Acyl)-N-(phenyl)]-amino-tetrahydro-2-furanon der Formel II

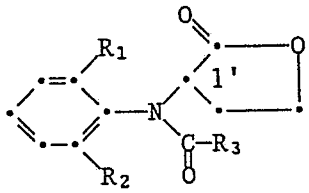

(II)

hydriert, das hydrierte Produkt durch Chlorierung in das Diastereomerengemisch (aR,1'R; aS,1'R) von den 3-[N-(Acyl)-N-(3-chlorphenyl)]-amino-tetrahydro-2-furanonen der Formel I überführt und daraus das Enantiomere (aS,1'R) durch Kristallisation aus einem Lösungsmittel isoliert.

Das (aS,1R)-Enantiomer zeigt eine erhöhte mikrobizide Aktivität gegenüber dem Isomerengemisch.

Das Zwischenprodukt der Formel

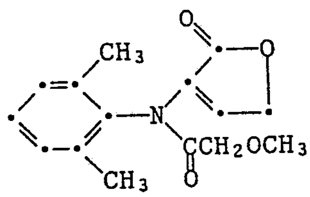

ist ebenfalls mikrobizid aktiv.

**Beschreibung**

**Verfahren zur Herstellung von substituierten Aniliden**

Die vorliegende Erfindung betrifft ein Verfahren zur Herstellung von mikrobizid wirksamen (aS;1′R)- und von (aR,1′R; aS,1′R)-3-[N-(Acyl)-N-(3-chlorphenyl)]-amino-tetrahydro-2-furanonen, neue, zum Teil mikrobizid wirksame Zwischenprodukte, mikrobizide Mittel, welche diese Aktivsubstanzen enthalten, sowie die Verwendung dieser Mittel als Mikrobizide im Pflanzenschutz.

Einige als Mikrobizide bekannte substituierte Anilide enthalten Asymmetriezentren und können dementsprechend als Stereoisomeren auftreten.

Das aus der DE-OS 2,804,299 (= GB-1,577,702) als Mikrobizid bekannte 3-[N-(Methoxyacetyl)-N-(3-chlor-2,6-dimethylphenyl)]-amino-tetrahydro-2-furanon der Formel Ia

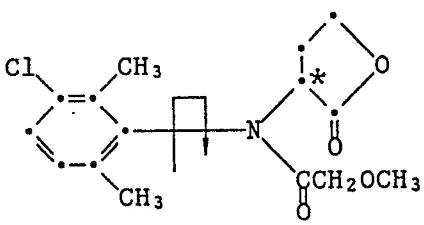

(Ia)

weist als Strukturmerkmal das mit * angedeutete Asymmetriezentrum auf. Es kann als Racemat auf übliche Art in optische Antipoden gespalten werden, wobei die verschiedenen Konfigurationen unterschiedlich starke mikrobizide Wirkung aufweisen.

Darüberhinaus werden in der DE-OS 2,804,299 im Rahmen des dortigen breiten chemischen Umfangs keine Angaben zu weiteren Isomerie-Möglichkeiten ausserhalb des Furanonrings gemacht.

Im Falle der konventionell als Racemat erhältlichen Verbindung der Formel I liegt aber nicht nur aufgrund der asymmetrischen Substitution am markierten *C-Atom ein einziges Enantiomerenpaar vor, sondern ein Gemisch von 4 Isomeren, d.h. von zwei diastereomeren Enantiomerenpaaren.

Der Grund hierfür liegt darin, dass das Molekül ausser dem erwähnten Asymmetriezentrum noch eine, durch die chirale Achse (P) bedingte Rotationsisomerie (Atropisomerie) aufweist, welche durch die asymmetrische Chlorsubstitution des rotationsbehinderten 2,6-Dimethylphenylrings verursacht wird. Das nach DE-OS 2,804,299 hergestellte 3-[N-(Methoxyacetyl)-N-(3-chlor-2,6-dimethylphenyl)]-amino-tetrahydro-2-furanon erweist sich als ein durch Adsorptionschromatographie oder Kristallisation trennbares Diastereomeren-Gemisch, das etwa je zu 50 % aus zwei bei 140° bzw. 115° schmelzenden diastereomeren Enantiomerenpaaren besteht. Diese beiden diastereomeren Enantiomerenpaare können auf übliche Art in optische Antipoden gespalten werden. Aus dem bei 140° C schmelzenden diastereomeren Enantiomerenpaar können die Enantiomeren (aS,1′S)- und (aR,1′R)-3-[N-(Methoxyacetyl)-N-(3-chlor-2,6-dimethylphenyl)]-amino-tetrahydro-2-furanone, aus dem bei 115° C schmelzenden diastereomeren Enantiomerenpaar die Enantiomeren (aR,1′S) und (aS,1′R), beispielsweise durch Chromatographie über eine optisch aktive Phase isoliert werden.

Das Enantiomere (aS,1′R)-3-[N-(Methoxyacetyl)-N-(3-chlor-2,6-dimethylphenyl)]-amino-tetrahydro-2-furanon weist eine sehr stark erhöhte mikrobizide Aktivität, verbunden mit einer unerwartet hohen Wirkungsdauer gegenüber dem Diastereomeren-Gemisch der Formel Ia auf.

Auch das durch Chlorierung des (1′R)-3-[(N-(Methoxyacetyl)-N-(2,6-dimethylphenyl)]-amino-tetrahydro-2-furanons[1] der Formel IIa

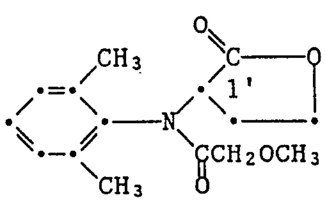

(IIa)

erhältliche Diastereomerengemisch (aR,1′R; aS,1′R)-3-[N-(Methoxyacetyl)-N-(3-chlor-2,6-dimethylphenyl)]-amino-tetrahydro-2-furanon zeigt eine wesentliche Erhöhung der mikrobiziden Aktivität gegenüber dem nach der DE-OS 2,804,299 erhältlichen Diastereomerengemisch, wobei das Ausmass der Wirkungssteigerung geringer ist, als beim (aS,1′R)-Enantiomeren.

Zur Herstellung des (aS,1′R) Enantiomers oder des Diastereomerengemisches (aR,1′R; aS,1′R) von 3-[N-(Methoxyacetyl)-N-(3-chlor-2,6-dimethylphenyl)]-amino-tetrahydro-2-furanon oder des (1′R)-3-[(N-(Methoxyacetyl)-N-(2,6-dimethylphenyl)]-amino-tetrahydro-2-furanons sind jedoch aus der Literatur keine Verfahren bekannt. Ziel der vorliegenden Erfindung ist deshalb die Bereitstellung eines überlegenen

[1] Das zum N nachbartständige asymmetrische C-Atom in der 3-Position des Lactonrings wird für die Konfigurationsangabe durchgängig mit (1′R) bzw. (1′S) bezeichnet.

Verfahrens zur Herstellung dieser Verbindungen.

Ein weiteres Ziel der vorliegenden Erfindung ist die Bereitstellung neuer mikrobizider Wirksubstanzen.

Es wurde gefunden, dass das Isomerengemisch (aS,1'R; aR,1'R) von 3-[N-(Methoxyacetyl)-N-(3-chlor-2,6-dimethylphenyl)]-amino-tetrahydro-2-furanon durch eine mehrstufige enantioselektive Synthese herstellbar ist und dass es in die beiden Enantiomeren (aS,1'R) und (aR,1'R) durch Kristallisation aufzutrennen ist.

Die Enantioselektivität ist bedingt durch eine, in Gegenwart von enantioselektiv wirkenden Katalysatoren verlaufende asymmetrische Hydrierung eines intermediären cyclischen Enamids. Enantioselektivität wird als die bevorzugte Bildung eines der beiden möglichen enantiomeren Reaktionsprodukte definiert, in diesem Falle der Reaktionsprodukte aus der Hydrierung.

Durch die Auswahl von geeigneten Katalysatoren wird die Entstehung des vom Gesichtspunkt der Erfindung aus wertvolleren Enantiomers bevorzugt.

Somit ist der Gegenstand der vorliegenden Erfindung ein Verfahren zur Herstellung von (aS;1'R)-3-[N-(Acyl)-N-(3-chlorphenyl)]-amino-tetrahydro-2-furanonen der Formel I

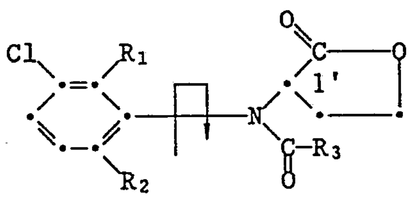

(I)

worin

$R_1$ und $R_2$ voneinander unabhänging Methyl oder Aethyl und

$R_3$ Methyl, Chlormethyl, Methoxymethyl oder Cyclopropyl bedeuten, wobei man

a) ein α-Phenylamino-γ-butyrolacton der Formel V

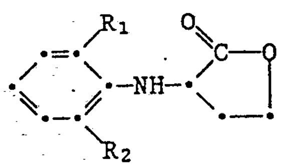

(V)

worin $R_1$ und $R_2$ die oben angegebene Bedeutung haben, zu einem 3-(Phenylamino)-2,5-dihydro-2-furanon der Formel IV

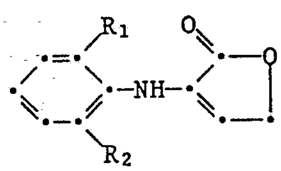

(IV)

worin $R_1$ und $R_2$ die oben angegebene Bedeutung haben, oxidiert, dieses

b) mit einem Carbonsäurehalogenid oder Carbonsäureanhydrid der Formel VI $R_3-\underset{\underset{O}{\|}}{C}-X$ (VI),

worin $R_3$ die unter der Formel I angegebene Bedeutung hat und X Chlor, Brom oder den Rest $R_3COO$-bedeutet, zu einem 3-[N-(Acyl)-N-(phenyl)]-amino-2,5-dihydro-2-furanon der Formel III

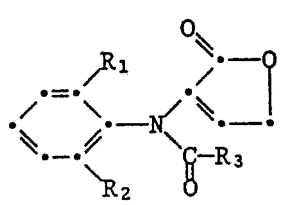

(III)

worin $R_1$, $R_2$ und $R_3$ die oben angegebene Bedeutung haben, acyliert,

c) das resultierende Produkt in Gegenwart von enantioselektiv wirkenden Katalysatoren mit Wasserstoff zu einem (1'R) 3-[N-(Acyl)-N-(phenyl)]-amino-tetrahydro-2-furanon der Formel II

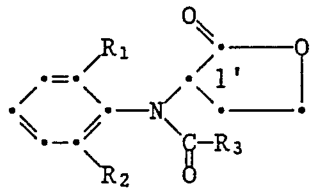

(II)

hydriert,

d) das hydrierte Produkt durch Chlorierung in das Diastereomerengemisch (aR,1′R; aS,1′R) von den 3-[N-(acyl)-N-(3-chlorphenyl)]-amino-tetrahydro-2-furanonen der Formel I überführt

e) und daraus das Enantiomere (aS,1′R) durch Kristallisation aus einem Lösungsmittel isoliert.

Die Erfindung betrifft ausser dem Gesamtverfahren auch deren einzelne Verfahrensschritte, wobei die Zwischenprodukte der Formeln II, III, IV und V gegebenenfalls isoliert werden können; sie betrifft aber auch die neuen Zwischenprodukte der Formeln III und IV. Das Zwischenprodukt der Formel IIIa

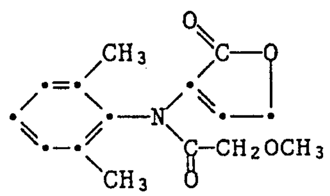

(IIIa)

besitzt mikrobizide Eigenschaften und ist deshalb auch als Wirksubstanz hervorzuheben.

Bevorzugt wird das Verfahren mit solchen Verbindungen der Formeln II bis VI ausgeführt, in welchen $R_1$ und $R_2$ Methyl, $R_3$ Methoxymethyl und X Chlor bedeuten. Daraus resultiert die Verbindung der Formel Ia.

Eine bevorzugte Ausführungsform der erfindungsgemässen Oxidation der Verbindungen der Formel V zu den Verbindungen der Formel IV besteht in der Umsetzung mit Mangandioxid als Oxidationsmittel in einem Lösungsmittel bei 40-140°C. Die Oxidation in Toluol bei 85-112°C, bzw. in Diäthylether wird dabei insbesondere bevorzugt.

Ebenfalls bevorzugt ist die Ausführungsform der Oxidation mit einer CerIV-Verbindung als Oxidationsmittel.

Eine weitere bevorzugte Ausführungsform der Oxidation der Verbindungen der Formel V erfolgt mit einem Peroxid oder einer Persäure als Oxidationsmittel in einem Lösungsmittel bei -10° bis +40°C unter atmospherischem Druck und führt zum entsprechenden [N-(Hydroxy)-N-(2,6-dialkylphenyl)]-amino-tetrahydro-2-furanon-Derivat. Letzteres wird durch Wasserabspaltung in die Verbindung der Formel IV überführt. Die Oxidation mit m-Chlorperbenzoesäure als Oxidationsmittel in Tetrahydrofuran bei 0-10°C wird dabei insbesondere bevorzugt. Die Wasserabspaltung kann durch Zusatz eines dehydratisierenden Mittels, wie beispielsweise basisches Aluminiumoxid erfolgen.

Bei der Durchführung der Oxidation mit m-Chlorperbenzoesäure im grösseren Massstab kann das Tetrahydrofuran durch andere geeignete Lösungsmittel vorteilhaft ersetzt werden. Als solche Lösungsmittel sind halogenierte Kohlenwasserstoffe, insbesondere halogenierte aliphatische Kohlenwasserstoffe, beispielsweise Dichlormethan zu nennen.

Die Acylierung der Verbindung der Formel IVa mit einem Ueberschuss von 50-500 % d.Th. der Verbindung der Formel VIa zur Verbindung der Formel IIIa, gegebenenfalls in Gegenwart eines Lösungsmittels wie z.B. Toluol, unter Abtrieb des als Nebenprodukt entstehenden Chlorwasserstoffs durch Sieden unter vermindertem Druck bei 40-60°C stellt eine bevorugte Ausführungsform der Acylierungs-Stufe des erfindungsgemässen Verfahrens dar.

Eine bevorzugte Ausführungsform der erfindungsgemässen enantioselektiven Hydrierung der Verbindung der Formel IIIa zum Enantiomeren (1′R) der Verbindung der Formel IIa erfolgt in Gegenwart katalytischer Mengen von Rhodiumverbindungen der Formeln VII oder VIIa

[XRhYZ] (VII) oder $[XRhY]^{\oplus}A^{\ominus}$ (VIIa)

worin X für zwei Olefinliganden oder einen Dienliganden steht, Y ein chirales Diphosphin, dessen sekundäre Phosphingruppen durch 2-4 C-Atome verknüpft sind und das zusammen mit dem Rh-Atom einen 5-, 6- oder 7-Ring bildet, oder Y ein chirales Diphosphinit, dessen Phosphinitgruppen über 2 C-Atome verknüpft sind und das zusammen mit dem Rh-Atom einen 7-Ring bildet, bedeutet, Z für Chlorid, Bromid oder Jodid steht, und $A^{\ominus}$ das Anion einer Sauerstoff- oder Komplexsäure darstellt, in einem Alkohol als Lösungsmittel, unter 0,1-150 bar bei-20 bis +70°C. Insbesondere bevorzugt ist dabei die Durchführung der Hydrierung in Gegenwart von je 0,002-10 Molprozent Bis-norbornadienyl-rhodium(I)-tetrafluoroborat und (4R,5R)-(-)-4,5-Bis-(diphenylphosphinomethyl)-2,2-dimethyl-1,3-dioxolan, bezogen auf die Verbindung der Formel IIIa, in Methanol unter 1-70 bar bei 0-50°C

Eine ebenfalls bevorzugte Ausführungsform dieser enantioselektiven Hydrierung erfolgt in Gegenwart von katalytischen Mengen eines Ru[(R)-2,2′-Bis(diarylphosphino)-1,1′-binaphthyl]-salzes der Formel VIIIa oder eines Ru[(S)-2,2′-Bis(diarylphosphino)-1,1′-binaphthyl]-salzes der Formel VIIIb

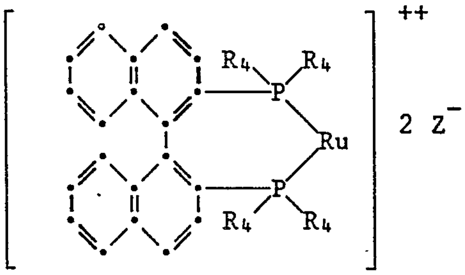

(VIIIa)

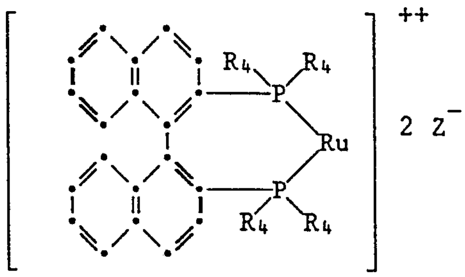

(VIIIb)

worin $R_4$ einen unsubstituierten oder durch $C_1$-$C_4$-Alkyl oder Methoxy monosubstituierten Phenylrest oder den 1- oder 2-Naphthylrest bedeutet und $Z^-$ entweder ein Anion der Formel $R_5COO^-$, worin $R_5$ für $C_1$-$C_6$-Alkyl oder $CF_3$ steht oder das Trifluormethansulfonat-Anion bedeutet, in einem Alkohol als Lösungsmittel, unter 0,1-150 bar bei -20 bis +70° C. Insbesondere bevorzugt ist dabei die Durchführung in Gegenwart von 0,002-10 Molprozent von Ru[(R)-oder (S)-2,2′-Bis(diphenylphosphino)-1,1′-binaphthyl]-diacetat, bezogen auf die Verbindung der Formel IIIa, in Methanol unter 1-70 bar bei 0-50°.

Als Chlorierungsmittel bei der Verbindungen der Formel II zu den Verbindungen der Formel I wird bevorzugt elementares Chlor eingesetzt.

Bei der Isolierung des (aS,1′R)-Enantiomeres der Formel Ia wird als Lösungsmittel Methylaethylketon bevorzugt.

Die vorliegende Erfindung umfasst auch ein Verfahren zur Herstellung des Diastereomerengemisches (aR,1′R; aS,1′R) im Verhältnis von 1:1 der Formel I, das sich von der soeben geschilderten Verfahrensweise einzig dadurch unterscheidet, dass das nach Chlorierung der Verbindung der Formel II entstehende Diastereomerengemisch anstelle der Abtrennung der aS,1′R Enantiomers, als Diastereomerengemisch isoliert wird. Bevorzugt wird dabei die Herstellung des Diastereomerengemisches (aR,1′R; aS,1′R) 3-[N-(Methoxyacetyl)-N-(3-chlor-2,6-dimethylphenyl)]-amino-tetrahydro-2-furanon der Formel Ia.

Ebenfalls Gegenstand der vorliegenden Erfindung bildet ein Verfahren zur Herstellung des Diastereomerengemisches (aS,1′R; aR,1′R) der Formel Ia durch Erwärmung des Enantiomers (aR,1′R) derselben Verbindung auf 160-180° C während 1-3 Stunden. Diese Verfahren wird erfindungsgemäss bevorzugt in Gegenwart von Tetrabutylammoniumbromid durchgeführt.

Die erfindungsgemässe Oxidation der bekannten Verbindungen der Formel V zu den Verbindungen der Formel IV, die auch als eine Dehydrierung eines Amins angesehen werden kann, ist ebenfalls neu, wobei die resultierenden Verbindungen der Formel IV ebenfalls neu sind. Auch die erfindungsgemässe Acylierung dieser Verbindungen mit den Carbonsäure-Derivaten der Formel VI zu den neuen Enamiden der Formel III ist noch nicht beschrieben worden. Als Carbonsäurederivat wird dabei Methoxyacetylchlorid bevorzugt. Die Acylierung kann auch wie üblich, in Gegenwart von äquivalenten Mengen von Basen, wie beispielsweise tert. Amine, durchgeführt werden. Die dabei zu erzielenden Ausbeuten sind jedoch mässig. Auch die Gegenwart von katalytischen Mengen von Carbonsäuredialkylamiden, wie beispielsweise Dimethylformamid anstelle der tert. Amine erhöht nur geringfügig die Ausbeute. Die besten Resultate können erfindungsgemäss in Abwesenheit von Basen oder Amiden mit überschüssigem Säurechlorid erzielt werden, wobei der als Nebenprodukt entstehende Chlorwasserstoff aus dem (gegebenenfalls unter vermindertem Druck) kochenden Reaktionsgut bei geeigneter Temperatur abgetrieben wird.

Die Zwischenprodukte der Formel IV sind neu und bilden ebenfalls Gegenstand der vorliegenden Erfindung. Die ebenfalls neuen Verbindungen der Formel III erwiesen sich überraschenderweise als Fungizide und sind ebenfalls Gegenstand der vorliegenden Erfindung. Bevorzugt wird dabei das 3-[N-(Methoxyacetyl)-N-(2,6-dimethylphenyl)]-amino-2,5-dihydro-2-furanon der Formel IIIa.

Die Ausgangsprodukte der Formeln V und VI des erfindungsgemässen Verfahrens sind bekannt oder analog zu bekannten Methoden herstellbar.

Im Mittelpunkt des erfindungsgemässen Verfahrens steht die enantioselektive Herstellung der Verbindungen der (1′R) 3-[N-(Acyl)-N-(phenyl)]-amino-tetrahydrofuran-2-furanonen der Formel II ausgehend aus den entsprechenden α-Phenyl-amino-γ-butyrolactonen der Formel V.

Hierzu wird dieses Lakton erfindungsgemäss zunächst zum entsprechenden 3-(Phenylamino)-2,5-dihydro-2-furanon der Formel IV oxidiert (bzw. dehydriert), wobei das Asymmetriezentrum in 3-Stellung am Furanring aufgehoben wird. Nach Acylierung des Enamids der Formel IV zum Enamid der Formel III wird letzteres erfindungsgemäss zum (1′R)-3-[N-(acyl)-N-(phenyl)]-amino-tetrahydro-2-furanon der Formel II hydriert. Als Resultat dieser asymmetrischen Hydrierung entsteht in 3-Stellung des Furanringes erneut ein Asymmetriezentrum, wobei die Bildung des unerwünschten 1′S Enantiomere zugunsten des erwünschten 1′R Enantiomere durch geeignete Massnahmen weitgehend unterdrückt wird.

Asymmetrische Hydrierung in Gegenwart von Rhodiumkatalysatoren wie sie auch unter den Formeln VII und VIIa definiert sind, oder in Gegenwart von Rutheniumverbindungen, wie sie unter den Formeln VIII und VIIIa

definiert sind, sind in der Literatur beschrieben.[2]

Die erfindungsgemässe asymmetrische Hydrierung der C=C-Doppelbindung der N-Phenyl-enamiden der Formel III ist neu.

Diese Hydrierungsvariante wird vorzugsweise in Gegenwart von jeweils 0,002-10 Mol% der Rhodiumkatalysatoren der Formeln VII oder VIIa oder der Rutheniumkatalysatoren der Formeln VIII oder VIIIa, bezogen auf die eingesetzten Verbindungen der Formel III, durchgeführt.

Die Reaktion wird bevorzugt in Lösungsmitteln, wie Alkohole, beispielsweise Methyl-, Aethyl-, Propyl- oder Butylalkohol; Kohlenwasserstoffe, beispielsweise Hexan, Toluol, Cyclohexan, Methylcyclohexan; Aether, beispielsweise Diäthyläther, Tetrahydrofuran, Dioxan; oder Ester, beispielsweise Aethylacetat, durchgeführt.

Die Katalysatoren der Formeln VII, VIIa sind aus der Literatur bekannt (siehe Uebersichtsliteratur). Das Bis-norbornadienyl-rhodium(I)-tetrafluoroborat und das (4R,5R)-(-)-4,5-Bis-(diphenylphosphinomethyl)-2,2-dimethyl-1,3-dioxolan sind im Handel erhältlich.

Die Katalysatoren der Formel VIII und VIIIa sind aus der Literatur ebenfalls bekannt (T. Okta, H. Takaya, M. Kitamura, K. Nagai, R. Noyori, J. Org. Chem. 1987, 52, 3174-3176; T. Okta, H. Takaya, R. Noyori, Inorg. Chem. 1988, 27, 566-569).

Die erfindungsgemässe asymmetrische Hydrierung wird unter Wasserstoffatmosphäre, vorzugsweise bei 0,1-150 bar Ueberdruck durchgeführt. Insbesondere wird dabei der Druckbereich von 1-70 bar bevorzugt. Als Reaktionstemperatur wird dabei der Bereich von -20 bis +70°C, insbesondere jener von 0 bis 50°C bevorzugt.

Aus den 3-[N-(Acyl)-N-(phenyl)]-amino-tetrahydro-2-furanonen können durch Chlorierung die Diastereomerengemische (aR,1'R; aS,1'R) von den 3-[N-(Acyl)-N-(3-chlorphenyl)]-amino-tetrahydro-2-furanonen der Formel I hergestellt werden, die entweder selbst als Mikrobizide verwendbar sind oder in die Enantiomeren (aR,1'R) und (aS,1'R) aufgetrennt werden können.

Das erfindungsgemässe Verfahren öffnet den vom technisch-wirtschaftlichen Gesichtspunkt aus interessanten Zugang zu hochwirksamen Stereoisomeren und deren Gemischen, insbesondere zum Diastereomerengemisch (aR,1'R; aS,1'R) und zum Enantiomeren (aS,1'R) des 3-[N-(acyl)-N-(3-chlor-2,6-dimethylphenyl)]-amino-tetrahydro-2-furanons.

Das als Nebenprodukt des erfindungsgemässen Verfahrens anfallende weniger wirksame Enantiomer (aR,1'R) kann erfindungsgemäss zum wertvollen Diastereomerengemisch (aR,1'R; aS,1'R) des 3-[N-(acyl)-N-(3-chlorphenyl)]-amino-tetrahydro-2-furanons thermisch umisomerisiert und als solches erneut in der geschilderten Weise eingesetzt werden (Rezyklierung).

In Ermangelung von enantioselektiven Herstellungsverfahren waren die hier erwähnten, wegen ihrer mikrobiziden Wirksamkeit hochwertigen Diastereomerengemische und Enantiomeren bis jetzt nur mit Hilfe von aufwendigen, für die technische Nutzung ungeeigneten Methoden herstellbar.

Bedeutend ist noch die Rahmen der vorliegenden Erfindung gemachte Feststellung der überraschenden mikrobiziden Wirksamkeit der Verbindungen der 3-(Phenylamino)-2,5-dihydro-2-furanone der Formel IV.

Das erfindungsgemässe Verfahren wird durch die folgenden Beispiele näher erläutert.

## Herstellungsbeispiele

### Beispiel H1: Herstellung des 3-[N-(2,6-Dimethylphenyl)]-amino-2,5-dihydro-2-furanons mit Mangandioxid als Oxidationsmittel

[Vergl. Journal of Organic Chemistry, 29, 1540 (1964)].

In eine 75°C warme Lösung von 53 g Kaliumpermanganat, in 330 g Wasser werden während 45 Minuten simultan 48,6 g Mangan(II)sulfat Monohydrat, gelöst in 84 ml Wasser, sowie 65 ml 40 % Natronlauge zugetropft und anschliessend 1 Stunde bei 80°C gerührt. Der Niederschlag wird abfiltriert, mit heissem Wasser neutral gewaschen und bei 125°C in Vakuum getrocknet. Die entstandenen 48,3 g Mangandioxid werden in 300 ml Toluol suspendiert und während 3 Stunden am Wasserabscheider gekocht. Dann werden 13,6 g 2-(2',6'-Dimethylphenylamino)-γ-butyrolacton zugegeben und über Nacht am Wasserabscheider gekocht. Die Lösung wird filtriert und eingedampft. Nach Chromatographie an Kieselgel mit Aether/Hexan 1:1 als Laufmittel erhält man 5,7 g (42 % d.Th.) 3-[N-(2,6-Dimethylphenyl)]-amino-2,5-dihydro-2-furanon als braune Kristalle (Smp. 80°C). Smp. nach Umkristallisation aus Aether/Hexan bei 84-85°C.

### Beispiel H2: Herstellung des 3-[N-(2,6-Dimethylphenyl)]-amino-2,5-dihydro-2-furanons mit Cer(IV)ammoniumnitrat als Oxidationsmittel

Zu einer Suspension von 3,47 g (6,3 mmol) Cer(IV)ammoniumnitrat in 20 ml Methylenchlorid werden 433 mg (2,1 mmol) 2-(2,5-Dimethylphenylamino)-γ-butyrolacton gegeben. Nach 18 stündigem Rühren bei Raumtemperatur wird die orange Reaktionsmischung filtriert und das Filtrat eingedampft. Nach einer Chromatographie des Rückstandes an Kieselgel mit Hexan/Essigester 2:1 als Laufmittel erhält man 190 mg (44 % d.Th.) 3-[N-(2,6-Dimethylphenyl)-]-amino-2,5-dihydro-2-furanon als gelb-braunes Kristallisat, das aus Hexan/Essige-

[2] Uebersichtsliteratur:
[1] K. Koenig, The applicability of asymmetric homogeneous catalytic hydrogenation in Asymmetric Synthesis, Vol. 5, S. Morrison (Ed.), Academic Press N.Y., Inc., 1985, pp. 71 ff.
[2] S. Halpern, Asymmetric catalytic hydrogenation, ibid, p. 41 ff,
[3] H.B. Kagan, Chiral Ligands for asymmetric catalysis , ibid, p. 13-23.)

ster 3:1 umkristallisiert wurde (Smp.: 85-86° C).

Beispiel H3a: Herstellung des 3-[N-(2,6-Dimethylphenyl)]-amino-2,5-dihydro-2-furanons mit m-Chlorperbenzoesäure als Oxidationsmittel

Zu einer Lösung von 61,5 g (0,3 Mol) 2-(2′,6′-Dimethylphenylamino)-γ-butyrolacton in 200 ml abs. Tetrahydrofuran wird bei 0° C innerhalb von 30-60 Minuten eine Lösung von 60,9 g (0,35 Mol) m-Chlorperbenzoesäure in 200 ml abs. Tetrahydrofuran gegeben. Das Reaktionsgut wird während 80 Minuten bei 0° C gerührt und durch 1 kg basisches Aluminiumoxid (ICN; Akt. I) unter Eiskühlung des Vorlage-Gefässes filtriert. Das Aluminiumoxid wird mit Tetrahydrofuran eluiert, die ersten 600 ml Eluat enthalten den Grossteil des gebildeten 3-[N-(2,6-Dimethylphenyl)]-amino-2,5-dihydro-2-furanon: 35,15 g in Form weisser Kristalle (57,6 % d.Th.). Smp. nach Umkristallisation aus Hexan 85-86° C.

Beispiel H3b: Herstellung des 3-[N-(2,6-Dimethylphenyl)-amino-2,5-dihydro-2-furanons mit m-Chlorperbenzoesäure als Oxidationsmittel

Zu einer Lösung von 61,5 g (0,3 Mol) 2-(2′,6′-Dimethylphenylamino)-γ-butyrolacton in 200 ml Dichlormethan wird bei 0° C innerhalb von 30-60 Minuten eine Lösung von 60,9 g (0,35 Mol) m-Chlorperbenzoesäure in 200 ml Dichlormethan gegeben. Das Reaktionsgut wird während 80 Minuten bei 0° C gerührt. Nach Filtrieren über wenig basisches Aluminiumoxid (ICN; Akt. I) wird das Lösungsmittel in Vakuum verdampft und der Rückstand in 400 ml Tetrahydrofuran gelöst. Die Lösung wird durch 1 kg basisches Aluminiumoxid unter Eiskühlung des Vorlage-Gefässes filtriert und analog zum Beispiel 3a aufgearbeitet. Resultat: 35,15 g Produkt in Form weisser Kristalle (57,6 % d.Th.). Smp. nach Umkristallisation aus Hexan 85-86° C.

Beispiel H4: Herstellung des 3-[N-(methoxyacetyl)-N-(2,6-dimethylphenyl)]-amino-2,5-dihydro-2-furanons

10,2 g (50,2 mmol) 3-[N-(2,6-Dimethylphenyl)]-amino-2,5-dihydro-2-furanon, 40 ml (439 mmol) Methoxyessigsäurechlorid und 40 ml Toluol werden zusammengegeben und bei 100-150 mbar während 48 Stunden unter Rückfluss bei 50-70° C gekocht. Anschliessend wird die Reaktionslösung eingedampft, der Rückstand in Hexan/Essigester-Gemisch (2:1) gelöst und an Kieselgel chromatographiert. Eluiert wird dabei mit einem Hexan/Essigester-Gemisch (1:2). Das nach Abdampfen des Lösungsmittels und zweimaliger Umkristallisation aus Hexan-Essigester-Gemisch (2:1) erhaltene 3-[N-(Methoxyacetyl)-N-(2,6-dimethylphenyl)]-amino-2,5-dihydro-2-furanon schmilzt bei 106-107° C.

Beispiel H5: Herstellung des 3-[N-(Methoxyacetyl)-N-(2,6-dimethylphenyl)]-amino-tetrahydro-2-furanons in Gegenwart von Bis-norbornadienyl-rhodium(I)-tetrafluoroborat und (4,5R)-(c)-4,5-Bis-(diphenylphosphinomethyl)-2,2-dimethyl-1,3-dioxolan

In einen 60 ml Stahlautoklav wird zunächst 0,9 g (4 mMol) 3-[N-(Methoxyacetyl)-N-(2,6-dimethylphenyl)]-amino-2,5-dihydro-2-furanon gelöst in 10 ml Methanol dann 29 mg (0,08 mMol) Bis-norbornadienyl-rhodium(I)- tetrafluoroborat und 44 mg (0,088 mMol) (4R,5R)-(-)-4,5-Bis(diphenylphosphinomethyl)-2,2-dimethyl-1,3-dioxolan, gelöst in 10 ml Methanol eingetragen. Die katalytische Hydrierung mit Wasserstoff wird bei 20-22° C mit einem Anfangsdruck von 33 bar Wasserstoff bis zum Aufnahmestillstand (etwa 1 Stunde) geführt.

Der nach Filtrieren und Eindampfen erhaltene Rückstand wird in Methylenchlorid/Methanol 98:2 gelöst und über Kieselgel chromatographiert. Das nach Verdampfen des Lösungsmittels erhältliche Produktgemisch fällt mit quantitativer Ausbeute an und besteht zu 90 % aus den Enantiomeren (1′R) 3-[N-(Methoxyacetyl)-N-(2,6-dimethylphenyl)]-amino-tetrahydro-2-furanon und zu 10 % aus dessen Antipoden.

Das reine (1′R) Enantiomer ist nach Umkristallisation aus Hexan/Ethylmethylketon 1:1 75 % Ausbeute rein erhältlich.

Beispiel H6: Herstellung des 3-[N-(Methoxyacetyl)-N-(2,6-dimethylphenyl)]-amino-tetrahydro-2-furanons in Gegenwart von Ru[(R)- oder (S)-2,2-Bis(diphenylphosphino]-1,1′-binaphthyl]-diacetat

In einen 60 ml Stahlautoklav wird zunächst 0,45 g (2 mMol) 3-[N-(Methoxyacetyl)-N-(2,6-dimethylphenyl)]-amino-2,5-dihydro-2-furanon gelöst in 10 ml Methanol dann 33.6 mg (0,04 mMol) Ru[(R)- oder (S)-2,2-Bis(diphenylphosphino)-1,1′-binaphthyl]-diacetat eingetragen. Die katalytische Hydrierung mit Wasserstoff wird bei 20-22° C mit einem Anfangsdruck von 30 bar Wasserstoff bis zum Aufnahmestillstand (etwa 1 Stunde) geführt.

Der nach Filtrieren und Eindampfen erhaltene Rückstand wird in Methylenchlorid/Methanol 98:2 gelöst und über Kieselgel chromatographiert. Das nach Verdampfen des Lösungsmittels erhältliche Produktgemisch fällt mit quantitativer Ausbeute an und besteht zu 83 % aus dem Enantiomeren (1′R) 3-[N-(Methoxyacetyl)-N-(2,6-dimethylphenyl)]-amino-tetrahydro-2-furanon und zu 17 % aus dessen Antipoden.

Das reine (1′R) Enantiomer ist nach Umkristallisation mit 65,7 % Ausbeute rein erhältlich.

Beispiel H7: Herstellung des Gemisches von (aR,1′R)-und (aS,1′R)-3-[N-(Methoxyacetyl)-N-(3-chlor-2,6-dimethylphenyl)]-amino-tetrahydro-2-furanon im Verhältnis von etwa 1:1

0,222 g (1′R) 3-[N-(Methoxyacetyl)-N-(2,6-dimethylphenyl)]-amino-tetrahydro-2-furanon werden in 30 ml Ameisensäure gelöst. 0,06 g Chlor werden bei Raumtemperatur in Gegenwart von 5 mg Eisen(III)chlorid in die Lösung eingeleitet. Die Lösung wird ca. 1 Std. bei Raumtemperatur gerührt und das Lösungsmittel unter

vermindertem Druck verdampft. Der Rückstand wird in Essigsäureäthylester gelöst, die Lösung mit Wasser extrahiert, mit Natriumsulfat getrocknet, filtriert und eingedampft. Das getrocknete Gemisch schmilzt bei 79-82°C, es enthält je ca. 50 % der beiden isomeren Titelprodukte.

Beispiel H8: Herstellung von (aS,1'R) 3-[N-(Methoxyacetyl)-N-(3-chlor-2,6-dimethylphenyl)]-amino-tetrahydro-2-furanon aus dem Gemisch (aR,1'R) und (aS,1'R) 3-[N-(Methoxyacetyl)-N-(3-chlor-2,6-dimethylphenyl)]-amino-tetrahydro-2-furanon im Verhältnis von etwa 1:1

0,5 g des 1:1 Gemisches (aR,1'R) und (aS,1'R) 3-[N-(Methoxyacetyl)-N-(3-chlor-2,6-dimethylphenyl)]-amino-tetrahydro-2-furanon werden in 0,25 g Methylaethylketon heiss gelöst, in der Kälte mit (aS,1'R)-3-[N-(Methoxyacetyl)-N-(3-chlor-2,6-dimethylphenyl)]-amino-tetrahydro-2-furanon angeimpft. Nach dreitägigem Stehen bei 0°C werden die ausgefallenen Kristalle abfiltriert und getrocknet. Das Kristallisat besteht aus reinem (aS,1'R)-3-[N-(Methoxyacetyl)-N-(3-chlor-2,6-dimethylphenyl)]-amino-tetrahydro-2-furanon.

Beispiel H9: Herstellung des 1:1 Gemisches (aR,1'R) und (aS,1'R) 3-[N-(Methoxyacetyl)-N-(3-chlor-2,6-dimethylphenyl)]-amino-tetrahydro-2-furanon aus dem reinen (aR,1'R) 3-[N-(Methoxyacetyl)-N-(3-chlor-2,6-dimethylphenyl)]-amino-tetrahydro-2-furanon

0,2 g (aR,1'R) 3-[N-(Methoxyacetyl)-N-(3-chlor-2,6-dimethylphenyl)]-amino-tetrahydro-2-furanon wird mit 0,01 g Tetrabutylammoniumbromid vermischt und 2 Stunden zwischen 160-180°C gehalten. Das nach Abkühlen kristallisierende Reaktionsgemisch wird mit 1 ml Wasser kalt verrieben, filtriert und getrocknet. Das mit quantitativer Ausbeute erhältliche Kristallisat besteht aus dem 1:1 Gemisch von (aR,1'R)- und (aS,1'R)-3-[N-(Methoxyacetyl)-N-(3-chlor-2,6-dimethylphenyl)]-amino-tetrahydro-2-furanon.

Formulierungsbeispiele (% = Gewichtsprozent)

F 1 Emulsion-Konzentrate

| | a) | b) | c) |
|---|---|---|---|
| Verbindung der Formel IIIa | 25% | 40% | 50% |
| Ca-Dodecylbenzolsulfonat | 5% | 8% | 6% |
| Ricinusöl-polyäthylenglykoläther (36 Mol Aethylenoxid | 5% | - | - |
| Tributylphenoyl-polyäthylenglykoläther (30 Mol Aethylenoxid) | - | 12% | 4% |
| Cyclohexanon | - | 15% | 20% |
| Xylolgemisch | 65% | 25% | 20% |

Aus solchen Konzentraten können durch Verdünnen mit Wasser Emulsionen jeder gewünschten Konzentration hergestellt werden.

F 2 Lösungen

| | a) | b) | c) | d) |
|---|---|---|---|---|
| Verbindung der Formel IIIa | 80% | 10% | 5% | 95% |
| Aethylenglykol-monomethyläther | 20% | - | - | - |
| Polyäthylenglykol M G 400 | - | 70% | - | - |
| N-Methyl-2-pyrrolidon | - | 20% | - | - |
| Epoxydiertes Kokosnussöl | - | - | 1% | 5% |
| Benzin (Siedegrenzen 160-190°C) | - | - | 94% | - |

(MG = Molekulargewicht)

Diese Lösungen eignen sich zur Herstellung von Mikrodispersionen.

F 3 Granulate

| | a) | b) |
|---|---|---|
| Verbindung der Formel IIIa | 5% | 10% |
| Kaolin | 94% | |
| Hochdisperse Kieselsäure | 1% | - |
| Attapulgit | - | 90% |

Der Wirkstoff wird in Methylenchlorid gelöst, auf den Träger aufgesprüht und das Lösungsmittel anschliessend im Vakuum abgedampft.

F 4 Stäubemittel

| | a) | b) |
|---|---|---|
| Verbindung der Formel IIIa | 2% | 5% |
| Hochdisperse Kieselsäure | 1% | 5% |
| Talkum | 97% | - |
| Kaolin | - | 90% |

Durch inniges Vermischen auf Trägerstoffe mit dem Wirkstoff erhält man gebrauchsfertige Stäubemittel.

F 5 Spritzpulver

| | a) | b) | c) |
|---|---|---|---|
| Verbindung der Formel IIIa | 25% | 50% | 75% |
| Na-Ligninsulfonat | 5% | 5% | - |
| Na-Laurylsulfat | 3% | - | 5% |
| Na-Diisobutylnaphthalinsulfonat | - | 6% | 10% |
| Octylphenolpolyäthylenglykoläther (7-8 Mol Aethylenoxid | - | 2% | - |
| Hochdisperse Kieselsäure | 5% | 10% | 10% |
| Kaolin | 62% | 27% | - |

Der Wirkstoff wird mit den Zusatzstoffen gut vermischt und in einer geeigneten Mühle vermahlen. Man erhält Spritzpulver, die sich mit Wasser zu Suspensionen jeder gewünschten Konzentration verdünnen lassen.

Biologische Beispiele

1. Wirkung gegen Phytophthora infestans auf Tomatenpflanzen

a) Residual-protektive Wirkung

Tomatenpflanzen wurden nach 3-wöchiger Anzucht mit einer aus Spritzpulver des Wirkstoffes, wie oben beschrieben, hergestellten Spritzbrühe (0,02 % Aktivsubstanz) besprüht. Nach 24 Stunden werden die behandelten Pflanzen mit einer Sporangiensuspension des Pilzes infiziert. Die Beurteilung des Pilzbefalls erfolgte nach einer Inkubation der infizierten Pflanzen während 5 Tagen bei 90-100 % Luftfeuchtigkeit und 20°C.

b) Systemische Wirkung

Zu Tomatenpflanzen wurde nach 3-wöchiger Anzucht eine aus Spritzpulver des Wirkstoffes, wie oben beschrieben, hergestellte Spritzbrühe gegossen [0,002 % Aktivsubstanz bezogen auf das Erdvolumen]. Es

wurde darauf geachtet, dass die Spritzbrühe nicht mit den oberirdischen Pflanzenteilen in Berührung kam. Nach 48 Stunden wurden die behandelten Pflanzen mit einer Sporangiensuspension des Pilzes infiziert. Die Beurteilung des Pilzbefalls erfolgte nach einer Inkubation der infizierten Pflanzen während 5 Tagen bei 90-100 % relativer Luftfeuchtigkeit und 20°C.

Die Verbindung der Formel IIIa erwies sich als voll wirksam: Der Pilzbefall betrug 0-5 %.

2. Plasmopara viticola auf Reben(Rebensämlinge, cv. Gutedel (Chasselas) 3 Töpfe mit je 1 Pflanze/Prüfsubstanz)

a) Residual protektive Blattapplikation

5 Wochen alte Rebensämlinge werden mit einer aus der formulierten Prüfsubstanz hergestellten Spritzbrühe besprüht und 1 Tag später mit einer Sporangiensuspension (200'000 Sprorangien/ml) von P. viticola infiziert. Die Inkubation erfolgt bei 20°C in einer Gewächshauskabine; einer 14-stündigen Inkubationsphase mit 100 % rel. Luftfeuchtigkeit folgen 4 Tage mit 75-85 % und abschliessend zur Induktion der Pilzsporulation noch eine Nacht mit 100 %. Nach der 6-tätigen Inkubation wird der Pilzbefall ausgewertet.

b) Systemische Wirkung

5 Wochen alte Rebensämlinge werden mit einer aus der formulierten Prüfsubstanz hergestellten Spritzbrühe angegossen. 3 Tage später werden die Pflanzen mit einer Sporensuspension (200'000 Sporangien/ml) von P. viticola infiziert. Ausführung des Versuchs wie unter a) beschrieben.

Mit der Verbindung der Formel IIIa wurde der Pilzbefall auf 0-5 % reduziert.

3. Systemische Wirkung gegen Pythium debaryanum an Zea mays

Der Pilz wurde auf Karottenschnitzel-Nährlösung kultiviert und einer Erde-Sand-Mischung beigegeben. Die so infizierte Erde wurde in Blumentöpfe abgefüllt und mit Maissamen besät. Gleich nach der Aussaat wurden die als Spritzpulver formulierten Versuchspräparate als wässrige Suspension in die Erde gegossen (20 ppm Wirkstoff der Formel IIIa bezogen auf das Erdvolumen). Die Töpfe wurden darauf während 3 Wochen im Gewächshaus bei ca. 20°C aufgestellt. Die Erde wurde dabei durch leichtes Ueberbrausen gleichmässig feucht gehalten. Bei der Auswertung der Tests wurde die Zahl der aufgelaufenen Maispflanzen sowie der Anteil gesunder und kranker Pflanzen bestimmt.

Die Zahl der aufgelaufenen und gesunden Pflanzen betrug mehr als 90 %.

**Patentansprüche**

1. Verfahren zur Herstellung von (aS;1'R)-3-[N-(Acyl)-N-(3-chlorphenyl)]-amino-tetrahydro-2-furanonen der Formel I

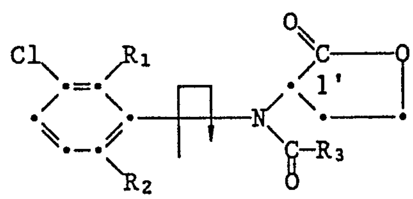

(I)

worin

$R_1$ und $R_2$ voneinander unabhängig Methyl oder Aethyl und

$R_3$ Methyl, Chlormethyl, Methoxymethyl oder Cyclopropyl bedeuten, dadurch gekennzeichnet, dass man

a) ein α-Phenylamino-γ-butyrolacton der Formel V

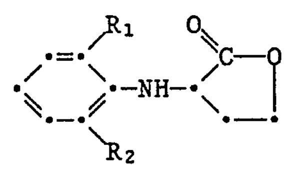

(V)

worin $R_1$ und $R_2$ die oben angegebene Bedeutung haben, zu einem 3-(Phenylamino)-2,5-dihydro-2-furanon der Formel IV

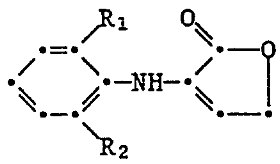

(IV)

worin $R_1$ und $R_2$ die oben angegebene Bedeutung haben, oxidiert, dieses

b) mit einem Carbonsäurehalogenid oder Carbonsäureanhydrid der Formel VI $R_3-\underset{\underset{O}{\|}}{C}-X$ (VI)

worin $R_3$ die unter der Formel I angegebene Bedeutung hat und X Chlor, Brom oder den Rest $R_3COO$- bedeutet, zu einem 3-[N-(Acyl)-N-(phenyl)]-amino-2,5-dihydro-2-furanon der Formel III

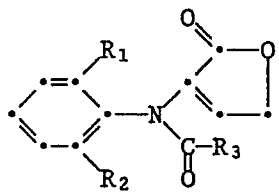

(III)

worin $R_1$, $R_2$ und $R_3$ die oben angegebene Bedeutung haben, acyliert,

c) das resultierende Produkt in Gegenwart von enantioselektiv wirkenden Katalysatoren mit Wasserstoff zu einem (1'R) 3-[N-(Acyl)-N-(phenyl)]-amino-tetrahydro-2-furanon der Formel II

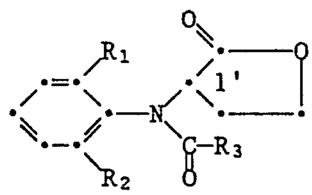

(II)

hydriert,

d) das hydrierte Produkt durch Chlorierung in das Diastereomerengemisch (aR,1'R; aS,1'R) von den 3-[N-(Acyl)-N-(3-chlorphenyl)]-amino-tetrahydro-2-furanonen der Formel I überführt

e) und daraus das Enantiomere (aS,1'R) durch Kristallisation aus einem Lösungsmittel isoliert.

2. Verfahren gemäss Anspruch 1, dadurch gekennzeichnet, dass $R_1$ und $R_2$ Methyl, $R_3$ Methoxymethyl und X Chlor bedeuten.

3. Verfahren gemäss Anspruch 2, dadurch gekennzeichnet, dass man die Oxidation der Verbindung der Formel Va in die Verbindung der Formel IVa

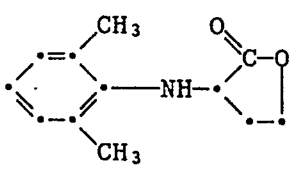

(Va)

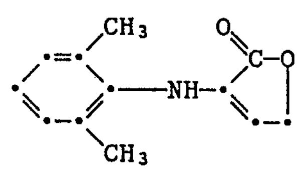

(IVa)

mit Mangandioxid als Oxidationsmittel in einem Lösungsmittel bei 40-140° C durchführt.

4. Verfahren gemäss Anspruch 3, dadurch gekennzeichnet, dass man die Oxidation in Toluol bei 85-112° C durchführt.

5. Verfahren gemäss Anspruch 2, dadurch gekennzeichnet, dass man die Oxidation mit einer CerIV-Verbindung als Oxidationsmittel durchführt.

6. Verfahren gemäss Anspruch 2, dadurch gekennzeichnet, dass man

a) die Verbindung der Formel Va, Anspruch 3, mit einem Peroxid oder einer Persäure in einem Lösungsmittel bei -10° bis +40° C unter atmosphärischem Druck zum [N-(Hydroxy)-N-(2,6-dimethylphenyl)]-amino-tetrahydro-2-furanon oxidiert und letzteres durch Wasserabspaltung in die Verbindung der Formel IVa überführt,

b) die Acylierung der Verbindung der Formel IVa mit einem Ueberschuss von 50-500 % d.Th. der Verbindung der Formel VIa zur Verbindung der Formel IIIa

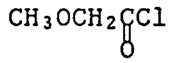

VIa

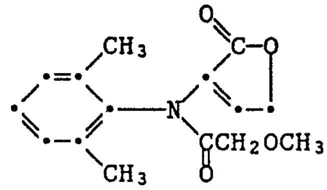

IIIa

unter Abtrieb des als Nebenprodukt entstehenden Chlorwasserstoffs durch Sieden unter vermindertem Druck bei 40-60° C,

c) die Hydrierung der Verbindung der Formel IIIa zum Enantiomeren (1'R) der Verbindung der Formel IIa

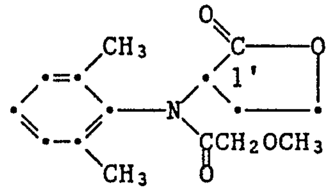

(IIa)

in Gegenwart katalytischer Mengen von Rhodiumverbindungen der Formeln VII oder VIIa

[XRhYZ] (VII) oder $[XRhY]^{\oplus}A^{\ominus}$ (VIIa)

worin X für zwei Olefinliganden oder einen Dienliganden steht, Y ein chirales Diphosphin, dessen sekundäre Phosphingruppen durch 2-4 C-Atome verknüpft sind und das zusammen mit dem Rh-Atom einen 5-, 6- oder 7-Ring bildet, oder Y ein chirales Diphosphinit, dessen Phosphinitgruppen über 2 C-Atome verknüpft sind und das zusammen mit dem Rh-Atom einen 7-Ring bildet, bedeutet, Z für Chlorid, Bromid oder Jodid steht, und $A^{\ominus}$ das Anion einer Sauerstoff- oder Komplexsäure darstellt, in einem Alkohol als Lösungsmittel, unter 0,1-150 bar bei -20° bis +70° C,

d) die Chlorierung der Verbindung der Formel IIa zur Verbindung der Formel Ia

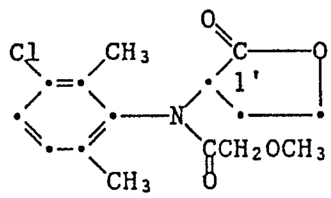

(Ia)

mit elementarem Chlor und

e) die Isolierung des Enantiomeren (aS,1'R) der Formel Ia aus Methylaethylketon als Lösungsmittel durchführt.

7. Verfahren gemäss Anspruch 6, dadurch gekennzeichnet, dass man die Oxidation mit m-Chlorperbenzoesäure als Oxidationsmittel in Tetrahydrofuran bie 0-10° C und die Hydrierung in Gegenwart von je 0,002-10 Molprozent Bis-norbornadienyl-rhodium(I)-tetrafluoroborat und (4R,5R)-(-)-4,5-Bis-(diphenylphosphinomethyl)-2,2-dimethyl-1,3-dioxolan, bezogen auf die Verbindung der Formel IIIa, in Methanol unter 1-70 bar bei 0-50° C durchführt.

8. Verfahren gemäss Anspruch 6, dadurch gekennzeichnet, dass man die Oxidation mit m-Chlorperbenzoesäure als Oxidationsmittel in Dichlormethan bei 0-10° C und die Hydrierung in Gegenwart von je 0,002-10 Molprozent Bis-norbornadienyl-rhodium(I)-tetrafluoroborat und (4R,5R)-(-)-4,5-Bis-(diphenylphosphinomethyl)-2,2-dimethyl-1,3-dioxolan, bezogen auf die Verbindung der Formel IIIa, in Methanol unter 1-70 bar bei 0-50° C durchführt.

9. Verfahren gemäss Anspruch 2, dadurch gekennzeichnet, dass man

a) die Verbindung der Formel Va, Anspruch 3, mit einem Peroxid oder einer Persäure in einem Lösungsmittel bei -10° bis +40° C unter atmosphärischem Druck zum N-[N-(Hydroxy)-N-(2,6-dimethylphenyl)]-amino-tetrahydro-2-furanon oxidiert und letzteres durch Wasserabspaltung in die Verbindung der Formel IVa überführt,

b) die Acylierung der Verbindung der Formel IVa mit einem Ueberschuss von 50-500 % d.Th. der Verbindung der Formel VIa zur Verbindung der Formel IIIa

$CH_3OCH_2COCl$

VIa

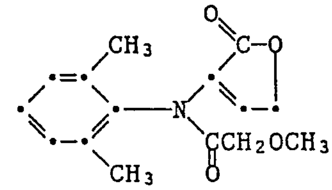

IIIa

unter Abtrieb des als Nebenprodukt entstehenden Chlorwasserstoffs durch Sieden unter vermindertem Druck bei 40-60° C, Hydrierung der Verbindung der Formel IIIa, Anspruch 6, zum Enantiomeren (1′R) der Verbindung der Formel IIa, Anspruch 6, in Gegenwart von katalytischen Mengen eines Ru[(R)-2,2′-Bis(diarylphosphino)-1,1′-binaphthyl]-salzes der Formel VIIIa oder eines Ru[(S)-2,2′-Bis(diarylphosphino)-1,1′-binaphthyl]-salzes der Formel VIIIb

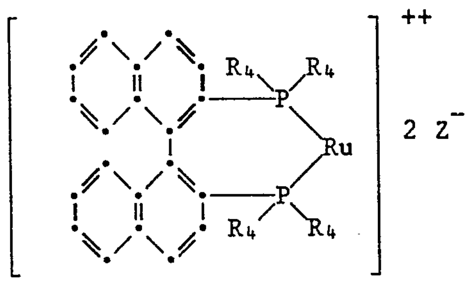

(VIIIa)

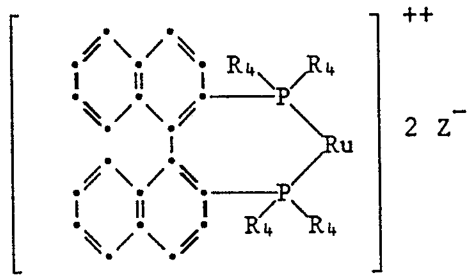

(VIIIb)

worin $R_4$ einen unsubstituierten oder durch $C_1$-$C_4$-Alkyl oder Methoxy monosubstituierten Phenylrest oder den 1- oder 2-Naphthylrest bedeutet und $Z^-$ entweder ein Anion der Formel $R_5COO^-$, worin $R_5$ für $C_1$-$C_6$-Alkyl oder $CF_3$ steht oder das Trifluormethansulfonat-Anion bedeutet, in einem Alkohol als Lösungsmittel, unter 0,1-150 bar bei -20 bis +70° C und die Chlorierung der Formel IIa zur Verbindung der Formel Ia, Anspruch 6, mit elementarem Chlor durchführt.

10. Verfahren gemäss Anspruch 9, dadurch gekennzeichnet, dass man die Hydrierung in Gegenwart von 0,002-10 Molprozent von Ru[(R)- oder (S)-2,2′-Bis(diphenylphosphino)-1,1′-binaphthyl]-diacetat, bezogen auf die Verbindung der Formel IIIa, in Methanol unter 1-70 bar bei 0-50° durchführt.

11. Verfahren zur Herstellung der Diastereomerengemische (aR,1′R; aS,1′R) von 3-[N-(Acyl)-N-(3-chlorphenyl)]-amino-tetrahydro-2-furanonen der Formel I, Anspruch 1, dadurch gekennzeichnet, dass man

a) ein 2-Phenyl-amino-γ-butyrolacton der Formel V, Anspruch 1 zu einem 3-(Phenyl-amino)-2,5-dihydro-2-oxofuran der Formel IV, Anspruch 1, oxidiert, dieses

b) mit einem Carbonsäurehalogenid oder Carbonsäureanhydrid der Formel VI, Anspruch 1, in ein 3-[N-(Methoxyacetyl)-N-(phenyl)]-amino-2,5-dihydro-2-furanon der Formel III, Anspruch 1 überführt,

c) das resultierende Produkt in Gegenwart eines selektiv wirkenden Katalysators mit Wasserstoff zum (1′R) 3-[N-(acyl)-N-(phenyl)]-amino-tetrahydro-2-furanon der Formel II, Anspruch 1 hydriert,

d) das hydrierte Produkt chloriert und das resultierende Diastereomerengemisch (aR,1′R; aS,1′R) der Verbindung der Formel I isoliert.

12. Verfahren gemäss Anspruch 11, dadurch gekennzeichnet, dass $R_1$ und $R_2$ Methyl, $R_3$ Methoxymethyl und X Chlor bedeuten.

13. Verfahren gemäss Anspruch 12, dadurch gekennzeichnet, dass man

a) die Oxidation der Verbindung der Formel Va, Anspruch 3 mit m-Chlorperbenzoesäure als Oxidationsmittel in Tetrahydrofuran bei 0-10° C durchführt und aus dem resultierenden N-Hydroxyderivat Wasser abspaltet,

b) die Acylierung der Verbindung der Formel IVa mit einem Ueberschuss von 50-500 % d.Th. der Verbindung der Formel VIa, Anspruch 6, zur Verbindung der Formel IIIa, Anspruch 6, unter Abtrieb des als Nebenprodukt entstehenden Chlorwasserstoffs durch Sieden unter vermindertem Druck bei 40-60° C,

c) die Hydrierung der Verbindung der Formel IIIa zum Enantiomeren (1′R) der Verbindung der Formel IIa, Anspruch 6, in Gegenwart katalytischer Mengen einer Rhodiumverbindung der Formel VII oder VIIa, Anspruch 6,

d) die Chlorierung der Formel IIa zur Verbindung der Formel Ia, Anspruch 6, mit elementarem Chlor durchführt.

14. Verfahren gemäss Anspruch 12, dadurch gekennzeichnet, dass man die Oxidation mit m-Chlorperbenzoesäure als Oxidationsmittel in Dichlormethan bei 0-10° C durchführt und aus dem resultierenden N-Hydroxyderivat Wasser abspaltet.

15. Verfahren gemäss den Ansprüchen 13 und 14, dadurch gekennzeichnet, dass man die Hydrierung in

Gegenwart von je 0,002-10 Molprozent Bis-norbornadienyl-rhodium(I)-tetrafluoroborat und von (4R,5R)-(-)-4,5-Bis-(diphenylphosphinomethyl)-2,2-dimethyl-1,3-dioxolan, bezogen auf die Verbindung der Formel IIIa, in Methanol unter 1-70 bar bei 0-50° C durchführt.

16. Verfahren gemäss Anspruch 12, dadurch gekennzeichnet, dass man

a) die Oxidation der Verbindung der Formel Va mit m-Chlorperbenzoesäure als Oxidationsmittel in Tetrahydrofuran bei 0-10° C durchführt und aus dem resultierenden N-Hydroxyderivat Wasser abspaltet,

b) die Acylierung der Verbindung der Formel IVa mit einem Ueberschuss von 50-500 % d.Th. der Verbindung der Formel VIa, Anspruch 6, zur Verbindung der Formel IIIa, Anspruch 6, unter Abtrieb des als Nebenprodukt entstehenden Chlorwasserstoffs durch Sieden unter vermindertem Druck bei 40-60° C,

c) die Hydrierung der Verbindung der Formel IIIa zum Enantiomeren (1'R) der Verbindung der Formel IIa, Anspruch 6, in Gegenwart katalytischer Mengen eines Ru[(R)-2,2'-Bis(diarylphosphino)-1,1'-binaphthyl]-salzes der Formel VIIIa oder eines Ru[(S)-2,2'-Bis(diarylphosphino)-1,1'-binaphthyl]-salzes der Formel VIIIb

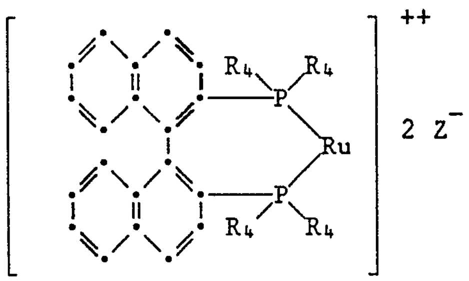

(VIIIa)

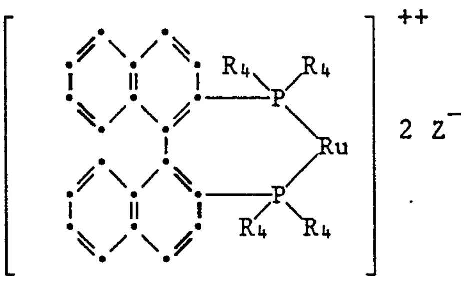

(VIIIb)

worin $R_4$ einen unsubstituierten oder durch $C_1$-$C_4$-Alkyl oder Methoxy monosubstituierten Phenylrest oder den 1- oder 2-Naphthylrest bedeutet und $Z^-$ entweder ein Anion der Formel $R_5COO^-$, worin $R_5$ für $C_1$-$C_6$-Alkyl oder $CF_3$ steht oder das Trifluormethansulfonat-Anion bedeutet, in einem Alkohol als Lösungsmittel, unter 0,1-150 bar bei -20 bis +70° C,

d) die Chlorierung der Verbindung der Formel IIa zur Verbindung der Formel Ia, Anspruch 6, mit elementarem Chlor durchführt.

17. Verfahren gemäss Anspruch 16, dadurch gekennzeichnet, dass man die Hydrierung in Gegenwart von 0,002-10 Molprozent Ru[(R)- oder (S)-2,2'-Bis(diphenylphosphino-1,1'-binaphthyl]-diacetat, bezogen auf die Verbindung der Formel IIIa, in einem Lösungsmittel unter 1-70 bar bei 0-50° durchführt.

18. Verfahren zur Herstellung des Diasteromerengemisches (aS,1'R; aR,1'R) der Verbindung der Formel I, Anspruch 1 aus dem Enantiomeren (aR,1'R) derselben Verbindung, dadurch gekennzeichnet, dass man letzteres während 1-3 Stunden auf 160-180° C erwärmt.

19. Verfahren nach Anspruch 18, dadurch gekennzeichnet, dass man die Erwärmung in Gegenwart von Tetrabutylammoniumbromid durchführt.

20. Verfahren zur Herstellung von 3-(Phenylamino)-2,5-dihydro-2-furanonen der Formel IV, Anspruch 1, dadurch gekennzeichnet, dass man ein 2-Phenylamino-γ-butyrolacton der Formel V, Anspruch 1 mit einem Oxidationsmittel oxidiert und das Reaktionsprodukt isoliert.

21. Verfahren nach Anspruch 20, dadurch gekennzeichnet, dass man die Oxidation mit m-Chlorperbenzoesäure als Oxidationsmittel in Tetrahydrofuran bie 0-10° C durchführt und aus dem resultierenden N-Hydroxyderivat Wasser abspaltet.

22. Verfahren nach Anspruch 21 zur Herstellung von 3-[N-(2,6-Dimethylphenyl)]-amino-2,5-dihydro-2-furanon der Formel IVa, Anspruch 3.

23. Verbindungen der Formel IV, Anspruch.

24. Das 3-[N-(2,6-Dimethylphenyl)]-amino-2,5-dihydro-2-furanon der Formel IVa, Anspruch 6, gemäss Anspruch 23.

25. Verfahren gemäss Anspruch 20, dadurch gekennzeichnet, dass man die Oxidation mit m-Chlorperbenzoesäure als Oxidationsmittel in Dichlormethan bei 0-10° C durchführt und aus dem resultierenden N-Hydroxyderivat Wasser abspaltet.

26. Verfahren zur Herstellung von 3-[N-(methoxyacetyl)-N-(phenyl)]-amino-2,5-dihydro-2-furanonen der Formel III, Anspruch 1, dadurch gekennzeichnet, dass man eine Verbindung der Formel IV, Anspruch 1 mit einer Verbindung der Formel VI, Anspruch 1 umsetzt und das Reaktionsprodukt isoliert.

27. Verfahren gemäss Anspruch 26, dadurch gekennzeichnet, dass $R_1$ und $R_2$ Methyl und X Chlor bedeuten.

28. Verfahren gemäss Anspruch 27, dadurch gekennzeichnet, dass man die Umsetzung mit einem Ueberschuss von 50-500 % d.Th. der Verbindung der Formel VIa unter Abtrieb des als Nebenprodukt entstehenden Chlorwasserstoffs durch Sieden unter vermindertem Druck, durchführt bei 40-60° C.

29. Verbindungen der Formel III, Anspruch 1.

30. Das 3-[N-(Methoxyacetyl)-N-(2,6-dimethylphenyl)]-amino-2,5-dihydro-2-furanon der Formel IIIa,

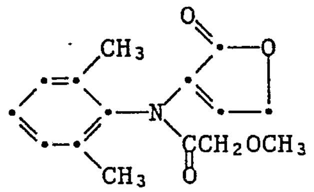

(IIIa)

gemäss Anspruch 29.

31. Verfahren zur Herstellung von (1'R)-3-[N-(Acyl)-N-(phenyl)]-amino-tetrahydro-2-furanon-Derivaten der Formel II, Anspruch 1, dadurch gekennzeichnet, dass man eine Verbindung der Formel III, Anspruch 1, in Gegenwart von selektiv wirkenden Katalysatoren mit Wasserstoff hydriert.

32. Verfahren gemäss Anspruch 31, dadurch gekennzeichnet, dass $R_1$ und $R_2$ Methyl und $R_3$ Methoxymethyl bedeuten.

33. Verfahren gemäss Anspruch 31, dadurch gekennzeichnet, dass man die Hydrierung in Gegenwart katalytischer Mengen einer Rhodiumverbindung der Formel VII oder VIIa, Anspruch 6, durchführt.

34. Verfahren gemäss Anspruch 33, dadurch gekennzeichnet, dass man die Hydrierung in Gegenwart von katalytischen Mengen von Bis-norbornadienylrhodium(I)-tetrafluoroborat und von (4R,5R)-(c)-4,5-Bis(diphenylphosphinomethyl)-2,2-dimethyl-1,3-dioxolan in einem Lösungsmittel, unter 1-70 bar bei 0-50° C durchführt.

35. Verfahren gemäss Anspruch 31, dadurch gekennzeichnet, dass man die Hydrierung in Gegenwart von katalytischen Mengen eines Ru[(R)- oder (S)-2,2'-Bis(diarylphosphino)-1,1'-binaphthyl]-salzes der Formeln VIIIa oder VIIIb

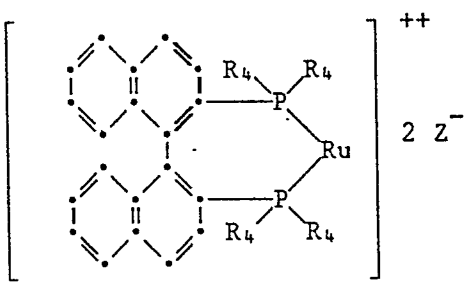

(VIIIa)

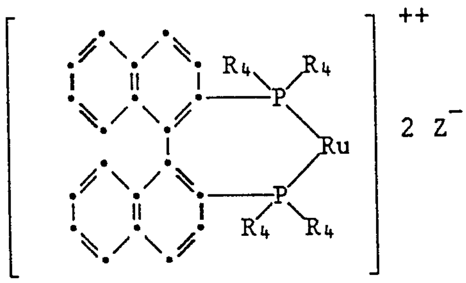

(VIIIb)

worin $R_4$ einen unsubstituierten oder durch $C_1$-$C_4$-Alkyl oder Methoxy monosubstituierten Phenylrest oder den 1- oder 2-Naphthylrest bedeutet und $Z^-$ entweder ein Anion der Formel $R_5COO^-$, worin $R_5$ für $C_1$-$C_6$-Alkyl oder $CF_3$ steht oder das Trifluormethansulfonat-Anion bedeutet, in einem Alkohol als Lösungsmittel, unter 0,1-150 bar bei -20 bis +70° C und die Chlorierung der Verbindung der Formel IIa zur Verbindung der Formel Ia, Anspruch 6, mit elementarem Chlor durchführt.

36. Verfahren gemäss Anspruch 35, dadurch gekennzeichnet, dass man die Hydrierung in Gegenwart von 0,002-10 Molprozent Ru[(R)- oder (S)-2,2'-Bis(diphenylphosphino)-1,1'-binaphthyl]-diacetat, bezogen auf die Verbindung der Formel IIIa, in einem Lösungsmittel unter 1-70 bar bei 0-50° durchführt.

37. Verfahren zur Herstellung des Diastereomerengemisches (aS,1'R;aR,1'R) der Formel I, Anspruch 1, dadurch gekennzeichnet, dass man die Verbindung der Formel II mit einem Chlorierungsmittel umsetzt.

38. Verfahren gemäss Anspruch 37, dadurch gekennzeichnet, dass man als Chlorierungsmittel Chlor verrwendet.

39. Fungizides Mittel enthaltend als Wirkstoff 0,1-95 % 3-[N-(Methoxyacetyl)-N-(2,6-dimethylphenyl)]-amino-2,5-dihydro-2-furanon der Formel IIIa, Anspruch 30, zusammen mit einem geeigneten Trägermaterial.

40. Verfahren zur Bekämpfung pflanzenschädigender Mikroorganismen, dadurch gekennzeichnet, dass man auf die Pflanze oder deren Standort eine mikrobizid wirksame Menge des 3-[N-(Methoxyacetyl)-N-(2,6-dimethylphenyl)]-amino-2,5-dihydro-2-furanons der Formel IIIa, Anspruch 30, appliziert.

**Patentansprüche für folgenden Vertragsstaat: ES**

1. Verfahren zur Herstellung von (aS;1'R)-3-[N-(Acyl)-N-(3-chlorphenyl)]-amino-tetrahydro-2-furanonen der Formel I

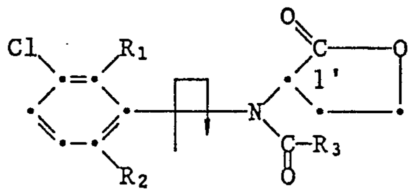

(I)

worin
$R_1$ und $R_2$ voneinander unabhängig Methyl oder Aethyl und
$R_3$ Methyl, Chlormethyl, Methoxymethyl oder Cyclopropyl bedeuten, dadurch gekennzeichnet, dass man

a) ein α-Phenylamino-γ-butyrolacton der Formel V

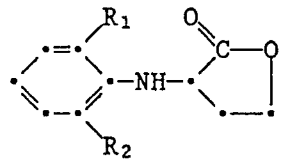

(V)

worin $R_1$ und $R_2$ die oben angegebene Bedeutung haben, zu einem 3-(Phenylamino)-2,5-dihydro-2-furanon der Formel IV

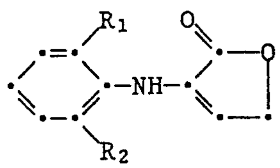

(IV)

worin $R_1$ und $R_2$ die oben angegebene Bedeutung haben, oxidiert, dieses

b) mit einem Carbonsäurehalogenid oder Carbonsäureanhydrid der Formel VI 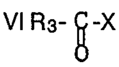 (VI)

worin $R_3$ die unter der Formel I angegebene Bedeutung hat und X Chlor, Brom oder den Rest $R_3COO$- bedeutet, zu einem 3-[N-(Acyl)-N-(phenyl)]-amino-2,5-dihydro-2-furanon der Formel III

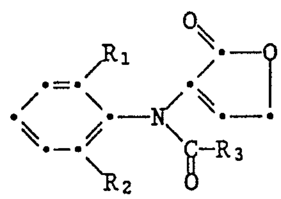

(III)

worin $R_1$, $R_2$ und $R_3$ die oben angegebene Bedeutung haben, acyliert,

c) das resultierende Produkt in Gegenwart von enantioselektiv wirkenden Katalysatoren mit Wasserstoff zu einem (1'R) 3-[N-(Acyl)-N-(phenyl)]-amino-tetrahydro-2-furanon der Formel II

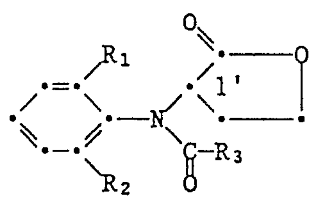

(II)

hydriert,

d) das hydrierte Produkt durch Chlorierung in das Diastereomerengemisch (aR,1'R; aS,1'R) von den 3-[N-(Acyl)-N-(3-chlorphenyl)]-amino-tetrahydro-2-furanonen der Formel I überführt

e) und daraus das Enantiomere (aS,1'R) durch Kristallisation aus einem Lösungsmittel isoliert.

2. Verfahren gemäss Anspruch 1, dadurch gekennzeichnet, dass $R_1$ und $R_2$ Methyl, $R_3$ Methoxymethyl und X Chlor bedeuten.

3. Verfahren gemäss Anspruch 2, dadurch gekennzeichnet, dass man die Oxidation der Verbindung der Formel Va in die Verbindung der Formel IVa

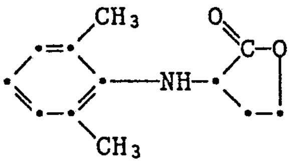

(Va)

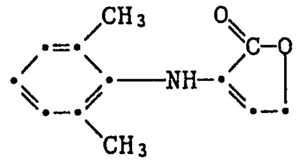

(IVa)

mit Mangandioxid als Oxidationsmittel in einem Lösungsmittel bei 40-140° C durchführt.

4. Verfahren gemäss Anspruch 3, dadurch gekennzeichnet, dass man die Oxidation in Toluol bei 85-112° C durchführt.

5. Verfahren gemäss Anspruch 2, dadurch gekennzeichnet, dass man die Oxidation mit einer CerIV-Verbindung als Oxidationsmittel durchführt.

6. Verfahren gemäss Anspruch 2, dadurch gekennzeichnet, dass man

a) die Verbindung der Formel Va, Anspruch 3, mit einem Peroxid oder einer Persäure in einem Lösungsmittel bei -10° bis +40° C unter atmosphärischem Druck zum [N-(Hydroxy)-N-(2,6-dimethylphenyl)-amino-tetrahydro-2-furanon oxidiert und letzteres durch Wasserabspaltung in die Verbindung der Formel IVa überführt,

b) die Acylierung der Verbindung der Formel IVa mit einem Ueberschuss von 50-500 % d.Th. der Verbindung der Formel VIa zur Verbindung der Formel IIIa

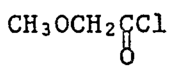

VIa

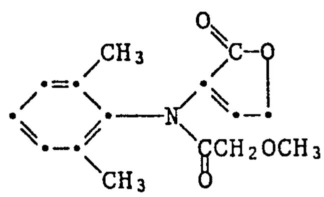

IIIa

unter Abtrieb des als Nebenprodukt entstehenden Chlorwasserstoffs durch Sieden unter vermindertem Druck bei 40-60° C,

c) die Hydrierung der Verbindung der Formel IIIa zum Enantiomeren (1'R) der Verbindung der Formel IIa

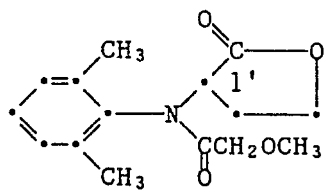

(IIa)

in Gegenwart katalytischer Mengen von Rhodiumverbindungen der Formeln VII oder VIIa

[XRhYZ] (VII) oder $[XRhY]^{\oplus}A^{\ominus}$ (VIIa)

worin X für zwei Olefinliganden oder einen Dienliganden steht, Y ein chirales Diphosphin, dessen sekundäre Phosphingruppen durch 2-4 C-Atome verknüpft sind und das zusammen mit dem Rh-Atom einen 5-, 6- oder 7-Ring bildet, oder Y ein chirales Diphosphinit, dessen Phosphinitgruppen über 2 C-Atome verknüpft sind und das zusammen mit dem Rh-Atom einen 7-Ring bildet, bedeutet, Z für Chlorid, Bromid oder Jodid steht, und $A^{\ominus}$ das Anion einer Sauerstoff- oder Komplexsäure darstellt, in einem Alkohol als Lösungsmittel, unter 0,1-150 bar bei -20° bis +70° C,

d) die Chlorierung der Verbindung der Formel IIa zur Verbindung der Formel Ia

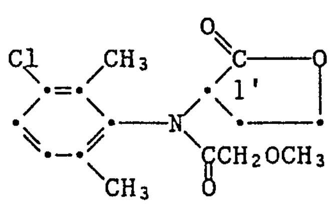

(Ia)

mit elementarem Chlor und

e) die Isolierung des Enantiomeren (aS,1'R) der Formel Ia aus Methylaethylketon als Lösungsmittel durchführt.

7. Verfahren gemäss Anspruch 6, dadurch gekennzeichnet, dass man die Oxidation mit m-Chlorperbenzoesäure als Oxidationsmittel in Tetrahydrofuran bei 0-10° C und die Hydrierung in Gegenwart von je 0,002-10 Mol prozent Bis-norbornadienyl-rhodium(I)-tetrafluoroborat und (4R,5R)-(-)-4,5-Bis-(diphenylphosphinomethyl)-2,2-dimethyl-1,3-dioxolan, bezogen auf die Verbindung der Formel IIIa, in Methanol unter 1-70 bar bei 0-50° C durchführt.

8. Verfahren gemäss Anspruch 6, dadurch gekennzeichnet, dass man die Oxidation mit m-Chlorperbenzoesäure als Oxidationsmittel in Dichlormethan bei 0-10° C und die Hydrierung in Gegenwart von je 0,002-10 Molprozent Bis-norbornadienyl-rhodium(I)-tetrafluoroborat und (4R,5R)-(-)-4,5-Bis-(diphenylphosphinomethyl)-2,2-dimethyl-1,3-dioxolan, bezogen auf die Verbindung der Formel IIIa, in Methanol unter 1-70 bar bei 0-50° C durchführt.

9. Verfahren gemäss Anspruch 2, dadurch gekennzeichnet, dass man

a) die Verbindung der Formel Va, Anspruch 3, mit einem Peroxid oder einer Persäure in einem Lösungsmittel bei -10° bis +40° C unter atmosphärischem Druck zum N-[N-(Hydroxy)-N-(2,6-dimethylphenyl)]-amino-tetrahydro-2-furanon oxidiert und letzteres durch Wasserabspaltung in die Verbindung der Formel IVa überführt,

b) die Acylierung der Verbindung der Formel IVa mit einem Ueberschuss von 50-500 % d.Th. der Verbindung der Formel VIa zur Verbindung der Formel IIIa

$CH_3OCH_2COCl$

VIa

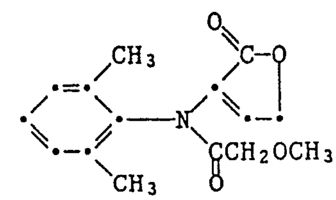

IIIa

unter Abtrieb des als Nebenprodukt entstehenden Chlorwasserstoffs durch Sieden unter vermindertem Druck bie 40-60° C, Hydrierung der Verbindung der Formel IIIa, Anspruch 6, zum Enantiomeren (1′R) der Verbindung der Formel IIa, Anspruch 6, in Gegenwart von katalytischen Mengen eines Ru[(R)-2,2′-Bis(diarylphosphino)-1,1′-binaphthyl]-salzes der Formel VIIIa oder eines Ru[(S)-2,2′-Bis(diarylphosphino)-1,1′-binaphthyl]-salzes der Formel VIIIb

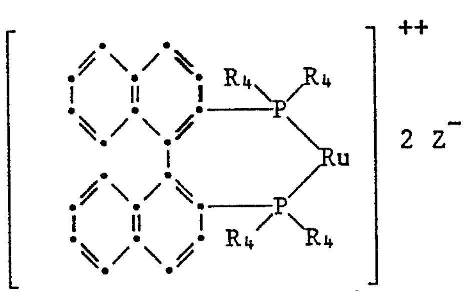

(VIIIa)

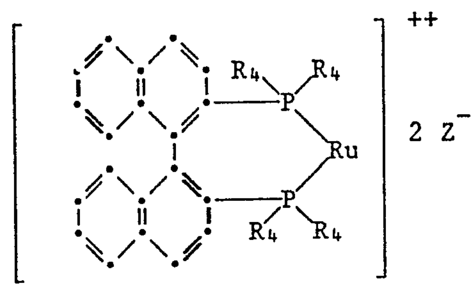

(VIIIb)

worin $R_4$ einen unsubstituierten oder durch $C_1$-$C_4$-Alkyl oder Methoxy monosubstituierten Phenylrest oder den 1- oder 2-Naphthylrest bedeutet und $Z^-$ entweder ein Anion der Formel $R_5COO^-$, worin $R_5$ für $C_1$-$C_6$-Alkyl oder $CF_3$ steht oder das Trifluormethansulfonat-Anion bedeutet, in einem Alkohol als Lösungsmittel, unter 0,1-150 bar bei -20 bis +70° C und die Chlorierung der Formel IIa zur Verbindung der Formel Ia, Anspruch 6, mit elementarem Chlor durchführt.

10. Verfahren gemäss Anspruch 9, dadurch gekennzeichnet, dass man die Hydrierung in Gegenwart von 0,002-10 Molprozent von Ru[(R)- oder (S)-2,2′-Bis(diphenylphosphino)-1,1′-binaphthyl]-diacetat, bezogen auf die Verbindung der Formel IIIa, in Methanol unter 1-70 bar bei 0-50° durchführt.

11. Verfahren zur Herstellung der Diastereomerengemische (aR,1′R; aS,1′R) von 3-[N-(Acyl)-N-(3-chlorphenyl)]-amino-tetrahydro-2-furanonen der Formel I, Anspruch 1, dadurch gekennzeichnet, dass man

a) ein 2-Phenyl-amino-γ-butyrolacton der Formel V, Anspruch 1 zu einem 3-(Phenyl-amino)-2,5-dihydro-2-oxofuran der Formel IV, Anspruch 1, oxidiert, dieses

b) mit einem Carbonsäurehalogenid oder Carbonsäureanhydrid der Formel VI, Anspruch 1, in ein 3-[N-(Methoxyacetyl)-N-(phenyl)]-amino-2,5-dihydro-2-furanon der Formel III, Anspruch 1 überführt,

c) das resultierende Produkt in Gegenwart eines selektiv wirkenden Katalysators mit Wasserstoff zum (1′R) 3-[N-(acyl)-N-(phenyl)]-amino-tetrahydro-2-furanon der Formel II, Anspruch 1 hydriert,

d) das hydrierte Produkt chloriert und das resultierende Diastereomerengemisch (aR,1′R; aS,1′R) der Verbindung der Formel I isoliert.

12. Verfahren gemäss Anspruch 11, dadurch gekennzeichnet, dass $R_1$ und $R_2$ Methyl, $R_3$ Methoxymethyl und X Chlor bedeuten.

13. Verfahren gemäss Anspruch 12, dadurch gekennzeichnet, dass man

a) die Oxidation der Verbindung der Formel Va, Anspruch 3 mit m-Chlorperbenzoesäure als Oxidationsmittel in Tetrahydrofuran bei 0-10° C durchführt und aus dem resultierenden N-Hydroxyderivat Wasser abspaltet,

b) die Acylierung der Verbindung der Formel IVa mit einem Ueberschuss von 50-500 % d.Th. der Verbindung der Formel VIa, Anspruch 6, zur Verbindung der Formel IIIa, Anspruch 6, unter Abtrieb des als Nebenprodukt entstehenden Chlorwasserstoffs durch Sieden unter vermindertem Druck bei 40-60° C,

c) die Hydrierung der Verbindung der Formel IIIa zum Enantiomeren (1'R) der Verbindung der Formel IIa, Anspruch 6, in Gegenwart katalytischer Mengen einer Rhodiumverbindung der Formel VII oder VIIa, Anspruch 6,

d) die Chlorierung der Formel IIa zur Verbindung der Formel Ia, Anspruch 6, mit elementarem Chlor durchführt.

14. Verfahren gemäss Anspruch 12, dadurch gekennzeichnet, dass man die Oxidation mit m-Chlorperbenzoesäure als Oxidationsmittel in Dichlormethan bei 0-10° C durchführt und aus dem resultierenden N-Hydroxyderivat Wasser abspaltet.

15. Verfahren gemäss den Ansprüchen 13 und 14, dadurch gekennzeichnet, dass man die Hydrierung in Gegenwart von je 0,002-10 Molprozent Bis-norbornadienyl-rhodium(I)-tetrafluoroborat und von (4R,5R)-(-)-4,5-Bis- (diphenylphosphinomethyl)-2,2-dimethyl-1,3-dioxolan, bezogen auf die Verbindung der Formel IIIa, in Methanol unter 1-70 bar bei 0-50° C durchführt.

16. Verfahren gemäss Anspruch 12, dadurch gekennzeichnet, dass man

a) die Oxidation der Verbindung der Formel Va mit m-Chlorperbenzoesäure als Oxidationsmittel in Tetrahydrofuran bei 0-10° C durchführt und aus dem resultierenden N-Hydroxyderivat Wasser abspaltet,

b) die Acylierung der Verbindung der Formel IVa mit einem Ueberschuss von 50-500 % d.Th. der Verbindung der Formel VIa, Anspruch 6, zur Verbindung der Formel IIIa, Anspruch 6, unter Abtrieb des als Nebenprodukt entstehenden Chlorwasserstoffs durch Sieden unter vermindertem Druck bei 40-60° C,

c) die Hydrierung der Verbindung der Formel IIIa zum Enantiomeren (1'R) der Verbindung der Formel IIa, Anspruch 6, in Gegenwart katalytischer Mengen eines Ru[(R)-2,2'-Bis(diarylphosphino)-1,1'-binaphthyl]-salzes der Formel VIIIa oder eines Ru[(S)-2,2'-Bis(diarylphosphino)-1,1'-binaphthyl]-salzes der Formel VIIIb

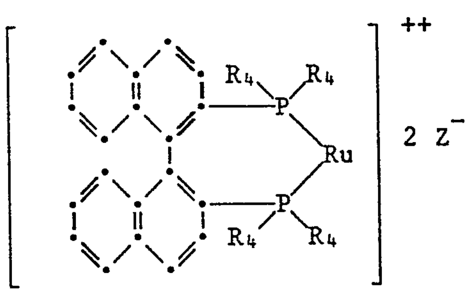

(VIIIa)

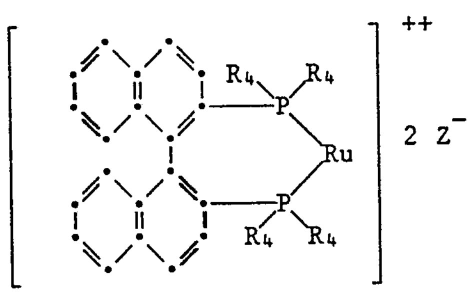

(VIIIb) .

worin $R_4$ einen unsubstituierten oder durch $C_1$-$C_4$-Alkyl oder Methoxy monosubstituierten Phenylrest oder den 1- oder 2-Naphthylrest bedeutet und $Z^-$ entweder ein Anion der Formel $R_5COO^-$, worin $R_5$ für $C_1$-$C_6$-Alkyl oder $CF_3$ steht oder das Trifluormethansulfonat-Anion bedeutet, in einem Alkohol als Lösungsmittel, unter 0,1-150 bar bei -20 bis +70° C,

d) die Chlorierung der Verbindung der Formel IIa zur Verbindung der Formel Ia, Anspruch 6, mit elementarem Chlor durchführt.

17. Verfahren gemäss Anspruch 16, dadurch gekennzeichnet, dass man die Hydrierung in Gegenwart von 0,002-10 Molprozent Ru[(R)- oder (S)-2,2'-Bis(diphenylphosphino-1,1'-binaphthyl]-diacetat, bezogen auf die Verbindung der Formel IIIa, in einem Lösungsmittel unter 1-70 bar bei 0-50° durchführt.

18. Verfahren zur Herstellung des Diasteromerengemisches (aS,1'R; aR,1'R) der Verbindung der Formel I, Anspruch 1 aus dem Enantiomeren (aR,1'R) derselben Verbindung, dadurch gekennzeichnet, dass man letzteres während 1-3 Stunden auf 160-180° C erwärmt.

19. Verfahren nach Anspruch 18, dadurch gekennzeichnet, dass man die Erwärmung in Gegenwart von Tetrabutylammoniumbromid durchführt.

20. Verfahren zur Herstellung von 3-(Phenylamino)-2,5-dihydro-2-furanonen der Formel IV, Anspruch 1, dadurch gekennzeichnet, dass man ein 2-Phenylamino-γ-butyrolacton der Formel V, Anspruch 1 mit einem Oxidationsmittel oxidiert und das Reaktionsprodukt isoliert.

21. Verfahren nach Anspruch 20, dadurch gekennzeichnet, dass man die Oxidation mit m-Chlorperbenzoesäure als Oxidationsmittel in Tetrahydrofuran bei 0-10° C durchführt und aus dem resultierenden N-Hydroxyderivat Wasser abspaltet.

22. Verfahren nach Anspruch 21 zur Herstellung von 3-[N-(2,6-Dimethylphenyl)]-amino-2,5-dihydro-2-furanon der Formel IVa, Anspruch 3.

23. Verfahren gemäss Anspruch 20, dadurch gekennzeichnet, dass man die Oxidation mit m-Chlorperbenzoesäure als Oxidationsmittel in Dichlormethan bei 0-10°C durchführt und aus dem resultierenden N-Hydroxyderivat Wasser abspaltet.

24. Verfahren zur Herstellung von 3-[N-(methoxyacetyl)-N-(phenyl)]-amino-2,5-dihydro-2-furanonen der Formel III, Anspruch 1, dadurch gekennzeichnet, dass man eine Verbindung der Formel IV, Anspruch 1 mit einer Verbindung der Formel VI, Anspruch 1 umsetzt und das Reaktionsprodukt isoliert.

25. Verfahren gemäss Anspruch 24, dadurch gekennzeichnet, dass $R_1$ und $R_2$ Methyl und X Chlor bedeuten.

26. Verfahren gemäss Anspruch 25, dadurch gekennzeichnet, dass man die Umsetzung mit einem Ueberschuss von 50-500 % d.Th. der Verbindung der Formel VIa unter Abtrieb des als Nebenprodukt entstehenden Chlorwasserstoffs durch Sieden unter vermindertem Druck, durchführt bei 40-60°C.

27. Verfahren zur Herstellung von (1′R)-3-[N-(Acyl)-N-(phenyl)]-amino-tetrahydro-2-furanon-Derivaten der Formel II, Anspruch 1, dadurch gekenzeichnet, dass man eine Verbindung der Formel III, Anspruch 1, in Gegenwart von selektiv wirkenden Katalysatoren mit Wasserstoff hydriert.

28. Verfahren gemäss Anspruch 27, dadurch gekennzeichnet, dass $R_1$ und $R_2$ Methyl und $R_3$ Methoxymethyl bedeuten.

29. Verfahren gemäss Anspruch 27, dadurch gekennzeichnet, dass man die Hydrierung in Gegenwart katalytischer Mengen einer Rhodiumverbindung der Formel VII oder VIIa, Anspruch 6, durchführt.

30. Verfahren gemäss Anspruch 29, dadurch gekennzeichnet, dass man die Hydrierung in Gegenwart von katalytischen Mengen von Bis-norbornadienylrhodium(I)-tetrafluoroborat und von (4R,5R)-(c)-4,5-Bis(diphenylphosphinomethyl)-2,2-dimethyl-1,3-dioxolan in einem Lösungsmittel, unter 1-70 bar bei 0-50°C durchführt.

31. Verfahren gemäss Anspruch 27, dadurch gekennzeichnet, dass man die Hydrierung in Gegenwart von katalytischen Mengen eines Ru[(R)- oder (S)-2,2′-Bis(diarylphosphino)-1,1′-binaphthyl]-salzes der Formeln VIIIa oder VIIIb

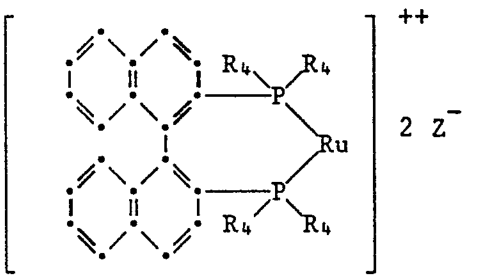

(VIIIa)

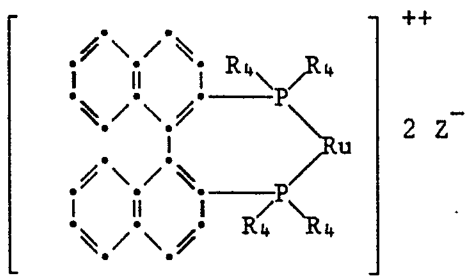

(VIIIb)

worin $R_4$ einen unsubstituierten oder durch $C_1$-$C_4$-Alkyl oder Methoxy monosubstituierten Phenylrest oder den 1- oder 2-Naphthylrest bedeutet und $Z^-$ entweder ein Anion der Formel $R_5COO^-$, worin $R_5$ für $C_1$-$C_6$-Alkyl oder $CF_3$ steht oder das Trifluormethansulfonat-Anion bedeutet, in einem Alkohol als Lösungsmittel, unter 0,1-150 bar bei -20 bis +70°C und die Chlorierung der Verbindung der Formel IIa zur Verbindung der Formel Ia, Anspruch 6, mit elementarem Chlor durchführt.

32. Verfahren gemäss Anspruch 31, dadurch gekennzeichnet, dass man die Hydrierung in Gegenwart von 0,002-10 Molprozent Ru[(R)- oder (S)-2,2′-Bis(diphenylphosphino)-1,1′-binaphthyl]-diacetat, bezogen auf die Verbindung der Formel IIIa, in einem Lösungsmittel unter 1-70 bar bei 0-50° durchführt.

33. Verfahren zur Herstellung des Diastereomerengemisches (aS,1′R; aR,1′R) der Formel I, Anspruch 1, dadurch gekennzeichnet, dass man die Verbindung der Formel II mit einem Chlorierungsmittel umsetzt.

34. Verfahren gemäss Anspruch 33, dadurch gekennzeichnet, dass man als Chlorierungsmittel Chlor verrwendet.

Europäisches Patentamt

# EUROPÄISCHER RECHERCHENBERICHT

Nummer der Anmeldung

EP 89 81 0450

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y,D | DE-A-2 804 299 (CIBA-GEIGY AG)<br>* Seite 1; Anspruch 1 *<br>--- | 1 | C 07 D 307/32<br>C 07 D 307/68<br>A 01 N 43/08 |
| Y | EP-A-0 077 755 (CIBA-GEIGY AG)<br>* Seite 14; Anspruch 1 *<br>--- | 1 | |
| Y | EP-A-0 218 970 (HOFFMANN-LA ROCHE & CO.)<br>* Seite 11, Anspruch 1; Seite 16, Anspruch 8 *<br>--- | 1 | |
| A | US-A-4 774 334 (V. S. GEORGIEV et al.)<br>* Spalte 4, Anspruch 1 *<br>--- | 23 | |
| Y | JOURNAL OF ORGANIC CHEMISTRY Band 52, Nr. 14, 10 Juli 1987, Seiten 3174-3176; G. W. GOKEL et al.: "Steroidal Lariat Ethers: A New Class of Macrocycles and the Crystal Structure of N-(Cholesterylosycarbonyl)aza-15-crown-5" * Seite 3175, Tabelle I, Spalte 1,2 *<br>--- | 1 | **RECHERCHIERTE SACHGEBIETE (Int. Cl.4)** |
| Y | JOURNAL OF ORGANIC CHEMISTRY Band 29, Nr. 6, 12 Juni 1964, Seiten 1540-1542; S. J. CRISTOL et al.: "Bridged Polycyclic Compounds. XXIV. The Halogenation of Substituted Norbornyl Radicals" * Seite 1540, Spalte 1 *<br>--- | 1 | C 07 D 307/00 |
| A | MIHALY NOGRADI: "STEREOSELECTIVE SYNTHESIS" Verlag Chemie Weinheim, 1986 * Seiten 57-80 *<br>----- | 1 | |

Der vorliegende Recherchenbericht wurde für alle Patentansprüche erstellt

| Recherchenort | Abschlußdatum der Recherche | Prüfer |
|---|---|---|
| BERLIN | 15-09-1989 | KYRIAKAKOU G |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)